# EUROPÄISCHE PATENTSCHRIFT

(11) **EP 0 671 151 B1**
(45) Veröffentlichungstag und Bekanntmachung des Hinweises auf die Patenterteilung: **09.02.2000**
(21) Anmeldenummer: 95102846.3
(22) Anmeldetag: 01.03.1995
(51) Int. Cl.: A61B 17/70

(54) **Osteosynthesevorrichtung**
Osteosynthetic device
Dispositif d'ostéosynthèse

(30) Priorität: 10.03.1994 DE 4407975; 18.03.1994 DE 4409242
(43) Veröffentlichungstag der Anmeldung: 13.09.1995
(73) Patentinhaber: SCHÄFER micomed GmbH, 73035 Göppingen (DE)
(72) Erfinder: Schäfer, Bernd, D-73035 Göppingen (DE); Zielke, Klaus, D-34537 Bad Wildungen (DE); Halm, Henry, Dr., 49143 Bissendorf-Wissingen (DE)
(74) Vertreter: Dreiss, Fuhlendorf, Steimle & Becker, Patentanwälte

(56) Entgegenhaltungen:
- EP-A- 0 348 272
- EP-A- 0 443 894
- EP-A- 0 452 792
- EP-A- 0 487 830
- WO-A-94/10927
- DE-A- 3 639 810
- FR-A- 2 624 720
- GB-A- 2 168 255
- US-A- 4 289 123
- US-A- 5 057 111

## Beschreibung

Die Erfindung betrifft eine Osteosynthesevorrichtung mit wenigstens zwei an einem Wirbel einer Wirbelsäule befestigbaren Knochenschrauben, wobei jede Knochenschraube einen Schraubenkopf aufweist, an dem bzw. mit dem ein Stab befestigbar ist und die beiden Stäbe über eine Querverbindungseinrichtung miteinander verbunden sind.

Derartige Osteosynthesevorrichtungen sind hinreichend bekannt. Sie dienen einerseits dazu, Knochen zu korrigieren, stabilisieren bzw. zu versteifen. Aus der DE 26 49 042 C2 ist z.B. eine Unterlagscheibe bekannt, die mittels einer Knochenschraube auf den Knochen aufgelegt und fixiert wird. Diese Knochenschraube ist zudem als Halter für einen Gewindestab ausgebildet, der als Korrekturstab dient. Die über den Gewindestab, die Knochenschraube und die Unterlagscheibe in den Knochen eingeleitete Kraft, die über die Unterlagscheibe auf den Knochen verteilt wird, bewirkt eine Lagekorrektur des Knochens. Ein derart korrigierter Knochen bedarf jedoch der Stabilisierung, wofür ein weiteres Stabilisierungselement am Knochen befestigt werden muß. Dieses Stabilisierungselement muß unter Umständen an einer anderen Stelle des Knochens befestigt werden, was eine weitere Operation erforderlich macht.

Weitere Osteosynthesevorrichtungen sind aus der DE 31 21 271 C2, DE 39 16 198 A1, DE 41 10 002 C1, DE 41 07 480 A1, EP 443 892 A1, CH 672 420 A5, GB 2,178,323 A, CA 1,304,267 A, US 5,030,220 A, DE 36 24 067 A1, EP 465 158 A2, EP 348 272 A1 und EP 346 521 A1 bekannt. In der letztgenannten Druckschrift ist deutlich erkennbar, daß einzelne Wirbel einer Wirbelsäule über eine Vielzahl von Knochenschrauben und zwei Stäbe miteinander verbunden sind. Auf diese Weise können die einzelnen Wirbel zwar korrigiert aber nicht stabilisiert werden. Zur Stabilisierung bedarf es zusätzlicher Elemente, die als Querverbinder zwischen den beiden Stäben angeordnet sind, wie es z.B. aus der EP 348 272 A1 bekannt ist. Derartige Systeme sind jedoch nur bedingt geeignet, die Lage einzelner Wirbel zu korrigieren, was in der Regel durch Verpressung aneinander anliegender Wirbel erfolgt.

Mit der US 4,289,123 A ist eine Vorrichtung bekannt geworden, mit der Wirbel über zwei Gewindestangen parallelverschoben werden können. Auch nur eine geringfügige Drehung einzelner Wirbel ist nicht möglich. Außerdem ist eine Stabilisierung der Wirbel nur bedingt möglich, da sie sich in der Ebene der Zwischenwirbelscheiben bewegen können.

Ausgehend von der EP 348 272 A1 liegt der Erfindung daher die Aufgabe zugrunde, eine einfache Osteosynthesevorrichtung bereitzustellen, mit der auf einfache Art und Weise eine dreidimensionale Korrektur z.B. einzelner Wirbel einer Wirbelsäule bzw. der Fehlstellung einzelner Wirbelkörper gegeneinander möglich ist. Die Osteosynthesevorrichtung soll außerdem primärstabil sein, d.h. eine erzielte Korrektur und Stabilisierung soll in der Regel ohne weitere innere und äußere Fixationsmechanismen belastungsstabil sein. Dies bedeutet, daß die auf diese Weise korrigierte und stabilisierte Wirbelsäule nach Abschluß der Wundheilung den physiologischen Anforderungen standhält.

Diese Aufgabe wird erfindungsgemäß mit einer Osteosynthesevorrichtung gelöst, die die Merkmale des Anspruchs 1 aufweist.

Dieser aktive Stab dient als Korrekturstab, über den die Lage und Ausrichtung der einzelnen Wirbel herbeigeführt wird. Der andere Stab dient ausschließlich zur Stabilisierung der korrigierten Lage der Wirbel, wobei die beiden Stäbe über die Querverbindungseinrichtung miteinander verbunden sind. Die Verbindung der beiden Stäbe über die Querverbindungseinrichtung hat den Zweck, daß die bei der Stabilisierung der Wirbel auftretenden Kräfte im wesentlichen auf den Stabilisierungsstab übertragen werden. Außerdem hat die Verankerung eines jeden Stabs direkt im Schraubenkopf der Knochenschraube den wesentlichen Vorteil, daß die Korrektur- und Stabilisierungskräfte direkt in den Knochen eingeleitet werden.

Bei einer erfindungsgemäßen Ausgestaltung ist die Querverbindungseinrichtung als Knochenplatte mit wenigstens zwei Befestigungsbohrungen zur Aufnahme der beiden Knochenschrauben ausgebildet. Die Korrektur- und Stabilisierungskräfte werden bei dieser Ausführungsform über die Knochenplatte zwischen den beiden Stäben übertragen, wobei die Platte außerdem die Verteilung der Zug-, Druck- und Schubkräfte sowie der Momente auf den Knochen übernimmt. Die erfindungsgemäße Osteosynthesevorrichtung ist geeignet, die einzelnen Knochen in weiten Bereichen in ihrer Fehlstellung gegeneinander zu korrigieren und gleichzeitig zu stabilisieren. Sie ist primärstabil. Nach der Applikation, die bevorzugt ventral erfolgt, bedarf es keiner weiteren Operation, z.B. für die Stabilisierung.

Bei einer Ausführungsform ist der aktive Stab als Gewindestab ausgebildet. Dieser Gewindestab ist bestens geeignet Korrekturkräfte gezielt auf einzelne Wirbelkörper zu übertragen. Außerdem kann der Gewindestab in Längsrichtung stufenlos verstellt werden.

Eine weitere Ausführungsform des aktiven Stabes sieht vor, daß dieser mit einer Rasterung versehen ist. Diese Rasterung in Form einer Zahn- oder Lochreihe, von Umfangsnuten oder dgl. gewährleistet, daß die Aktivkräfte zur Mobilisierung der Wirbelkörper sicher in den Stab eingeleitet und von diesem auf die Wirbelkörper übertragen werden können. Eine Lagekorrektur kann z.B. auch durch Verkantung von Feststellelementen am aktivken Stab erreicht werden.

Vorteilhaft ist z.B. der Gewindestab über zwei Muttern am Schraubenkopf der Knochenschraube befestigbar. Dieses System ist an sich bei Knochenschrauben bekannt. Über derartige Knochenschrauben werden sowohl Knochenplatten am Knochen befestigt als auch ein Gewindestab festgelegt. Über die Muttern kann der Gewindestab gezielt verlagert und dadurch die einzelnen Knochen bzw. Wirbel aktiv korrigiert und auch gedreht bzw. verschwenkt oder verschoben werden. Der aktive bzw. Gewindestab bildet den dynamischen Teil der Osteosynthesevorrichtung.

Gemäß einem bevorzugten Ausführungsbeispiel weisen der Schraubenkopf in Achsrichtung des Gewindestabs um die Aufnahmeöffnung für den Gewindestab sich erstreckende Vorsprünge und die Mutter die Vorsprünge aufnehmende Ausnehmungen auf. Bei einem anderen Ausführungsbeispiel weisen der Schraubenkopf in Achsrichtung des Gewindestabs um die Aufnahmeöffnung für den Gewindestab sich erstreckende Ausnehmungen und die Mutter in die Ausnehmungen eingreifende Vorsprünge auf. Die Verankerung der Muttern am Schraubenkopf über Vorsprünge und Ausnehmungen hat den wesentlichen Vorteil, daß der Gewindestab sicher im Schraubenkopf, der gabelförmig ausgebildet, nicht nur in Axialrichtung festgelegt ist, sondern auch gegen ein Herausrutschen gesichert wird. Dabei können die Ausnehmungen und Vorsprünge im wesentlichen kegelstumpfförmig, rechteckförmig oder kugelförmig ausgebildet sein. Die Ausnehmung ist stets konkav und der Vorsprung stets konvex doch jeweils kongruent gestaltet.

Bei einer Weiterbildung ist vorgesehen, daß der Schraubenkopf ein Außengewinde für eine aufschraubbare Fixiereinrichtung aufweist. Diese Fixiereinrichtung verschließt den Gabelkopf und sichert dadurch den eingelegten Stab einerseits gegen eine axiale Verlagerung, andererseits gegen ein Verdrehen im Lager des Gabelkopfes. Selbstverständlich verhindert die Fixiereinrichtung auch ein Herausfallen des Stabes aus dem Gabelkopf.

Ein bevorzugtes Ausführungsbeispiel sieht vor, daß die Fixiereinrichtung als Überwurfmutter oder als Hutmutter bzw. Kopfmutter ausgebildet ist. Auf diese Weise werden sowohl die beiden Schenkel des Gabelkopfes gegen eine Aufspreizung gesichert, als auch der Gabelkopf auf einfache Weise verschlossen und der darin eingelegte Stab sicher gehalten. Andere Fixiereinrichtung sind denkbar. Bevorzugt ist die Hutmutter mit einer koaxial einschraubbaren Fixierschraube, insbesondere mit einem Innensechskant und/oder einer Ringschneide, versehen. Über den Innensechskant kann nach dem Aufschrauben der Hutmutter die Fixierschraube verstellt und derart auf den eingelegten Stab aufgeschraubt werden, daß sich die Ringschneide in das Material des Fixierstabes eingräbt. Auf diese Weise wird eine Sicherung sowohl gegen axiales Verschieben als auch gegen Verdrehen geschaffen.

Bevorzugt ist einer der Stäbe als längsgenuteter Stab ausgebildet. Außerdem kann der Schraubenkopf einer Knochenschraube derart ausgebildet sein, daß der Grund des Gabelkopfes mit hinterschneidungsfreien Längskerben versehen ist, in die der mit den Längsnuten versehene Stab eingesetzt werden kann. Durch die Längskerben im Gabelkopf und die Längsnuten im Stab wird eine zusätzliche Verdrehsicherung dieses Stabes um seine Längsachse geschaffen. Dieser Stab bildet den statischen bzw. passiven Teil der Osteosynthesevorrichtung.

Gemäß einem bevorzugten Ausführungsbeispiel sind die Durchmesser der Stäbe unterschiedlich groß, insbesondere ist der Durchmesser des Gewindestabes kleiner als der des anderen Stabs. Durch den aktiven Stab mit kleinerem Durchmesser wird auf einfache Weise dessen Flexibilität gegenüber dem anderen Stab erhöht, so daß mit dem aktivem Stab problemlos durch dessen Verformung die Lage der einzelnen Wirbelkörper zueinander korrigiert werden kann. Eine hohe Flexibilität kann auch durch die Wahl eines geeigneten zugelassenen Materials, ob Metall oder Kunststoff oder einer Kombination erzielt werden.

Vorteilhaft ist der Gewindestab ein Korrekturstab und der andere Stab dient zur Stabilisierung des Systems. Es wird also zuerst über den Gewindestab die gewünschte Lage der einzelnen Knochen eingestellt und diese Lage anschließend mit dem anderen Stab fixiert bzw. stabilisiert. Dadurch ist das erfindungsgemäße System primärstabil.

Bevorzugt verlaufen die Befestigungsbohrungen orthogonal zur Knochenplatte und/oder schräg zu dieser. Auf diese Weise konvergieren oder divergieren die Knochenschrauben in die gewünschte Richtung aufgrund der an die Wölbung der Knochenoberfläche angefaßten Knochenplatte, so daß die Knochenplatte bzw. die Osteosynthesevorrichtung auch bei relativ kleinen Knochen noch sicher befestigbar ist. Vorteilhaft kann nun die Knochenschraube derart in den Wirbel eingeschraubt werden, daß ein genügend großer Abstand zum Knochenmark besteht und daß die gerundete Spitze der Schraube und ein Gewindegang auf der anderen Seite des Knochens wieder auftreten.

Erfindungsgemäß weisen die Befestigungsbohrungen jeweils ein kalottenförmiges Lager für den Schraubenkopf der Knochenschraube auf. Hierdurch wird die Möglichkeit geschaffen, daß die Knochenschraube nicht ausschließlich orthogonal zur Knochenplatte eingeschraubt werden muß, sondern eine geringe Schrägstellung einnehmen kann, so daß der Schraubenschaft unabhängig von der Lage der Knochenplatte in Bereiche des Knochens eingedreht werden kann, in denen er einen optimalen Halt finden kann. Außerdem dient das kalottenförmige Lager zusätzlich zur Aufnahme von Querkräften von der Knochenschraube auf die Knochenplatte bzw. umgekehrt.

Bevorzugt sind die beiden Stäbe im wesentlichen parallel zueinander angeordnet. Dies ist insbesondere bei der Verwendung der Osteosynthesevorrichtung an Wirbelsäulen von Vorteil, da auf diese Weise mehrere Knochenplatten hintereinander an jeweils einem Wirbel befestigt werden können.

Weitere Vorteile, Merkmale und Einzelheiten der Erfindung ergeben sich aus der nachfolgenden Beschreibung, in der unter Bezugnahme auf die Zeichnung ein besonders bevorzugtes Ausführungsbeispiel im einzelnen dargestellt ist. Dabei können die in der Zeichnung dargestellten und in der Beschreibung erwähnten Merkmale jeweils einzeln für sich oder in einer beliebigen Kombination bei der Erfindung verwirklicht sein. In der Zeichnung zeigen:
- Figur 1: einen Querschnitt durch eine Knochenplatte mit zwei in die Knochenplatte eingesetzten Knochenschrauben;
- Figur 2: eine Seitenansicht der in der Figur 1 rechts dargestellten Knochenschraube;
- Figur 3: eine Draufsicht auf die Knochenschraube der Figur 2;
- Figur 4: eine Explosionsdarstellung des oberen Teils der in Figur 1 links dargestellten Knochenschraube mit einzulegendem Stab, Hutmutter und Fixierschraube;
- Figur 5: eine Draufsicht auf die Knochenplatte gemäß Figur 1; und
- Figur 6: eine schematische Darstellung der Verlagerung eines Wirbelknochens.

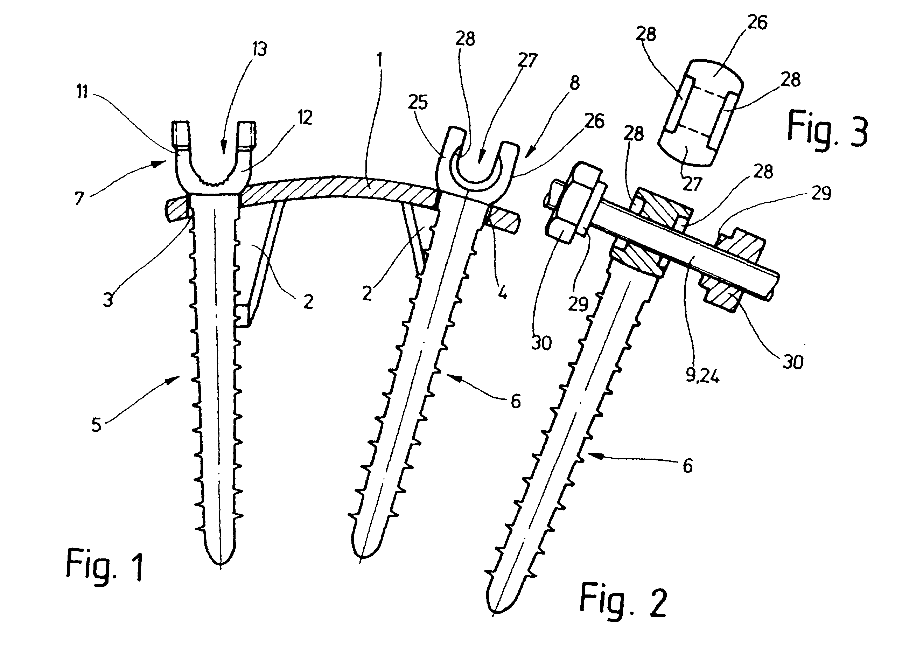

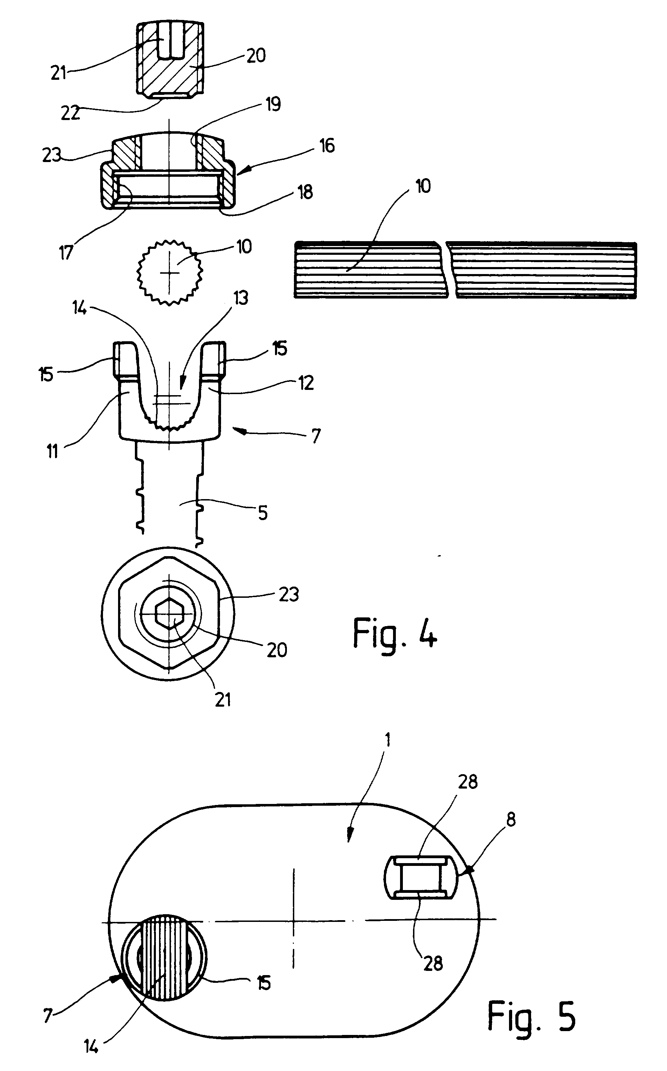

In der Figur 1 ist eine mit 1 bezeichnete Knochenplatte andeutungsweise dargestellt. An der proximalen Seite (Unterseite) der Knochenplatte 1 sind mehrere Ankerstifte 2 vorgesehen, von denen in der Figur 1 lediglich zwei teilweise verdeckt dargestellt sind. Über die Ankerstifte 2, die in den Knochen eingeschlagen werden, kann die Knochenplatte 1 verschiebesicher am Knochen verankert werden. Ferner ist in Figur 1 erkennbar, daß die Knochenplatte 1 zwei Befestigungsbohrungen 3 und 4 aufweist, in die jeweils eine Knochenschraube 5 und 6 eingesetzt ist. Bevorzugt sind die beiden Befestigungsbohrungen 3 und 4 in Bezug auf des Zentrums der Knochenplatte 1 einander gegenüberliegend angeordnet, was aus Figur 5 erkennbar ist. Die Befestigungsbohrungen 3 und 4 können ein kalottenförmiges Lager aufweisen, in dem der untere Teil des Schraubenkopfes 7 bzw. 8 der Knochenschraube 5 bzw. 6 gelagert ist. Dieser untere Teil des Schraubenkopfes 5 bzw. 8 ist dann entsprechend sphärisch ausgebildet, so daß er formschlüssig von diesem Lager aufgenommen wird. Ferner ist in Figur 2 erkennbar, daß die rechte Knochenschraube 6 im wesentlichen orthogonal zur Oberfläche der Knochenplatte 1, d.h. im wesentlichen radial zu deren Krümmung ausgerichtet ist. Die Knochenschraube 5 ist schräggestellt und erstreckt sich im wesentlichen parallel zur Symmetrieachse der Knochenplatte 1.

Die beiden Knochenschrauben 5 und 6 sind mit unterschiedlichen Schraubenköpfen 7 und 8 versehen, so daß unterschiedliche Stäbe 9 und 10 (Figuren 2 und 4) aufgenommen werden können. Die in der Figur 1 links dargestellte Knochenschraube 5 ist mit einem gabelförmigen Schraubenkopf 7 versehen, der zwei Schenkel 11 und 12 aufweist. Dieser gabelförmige Schraubenkopf 7 ist in Figur 4 näher dargestellt. Die beiden Schenkel 11 und 12 bilden zwischen sich eine Aufnahmeöffnung 13 für den Stab 10, der als längsgenuteter Stab ausgebildet ist. Der Grund der Aufnahmeöffnung 13 ist mit Längskerben 14, die hinterschneidungsfrei sind, versehen, in die der Stab 10 eingelegt werden kann. Die Längskerben 14 korrespondieren mit den Längsnuten des Stabes 10 und verhindern eine Verdrehung des Stabes 10 in der Aufnahmeöffnung 13. Zur Fixierung des Stabes 10 in der Aufnahmeöffnung sind die beiden Schenkel 11 und 12 an ihrer Außenseite mit einem Außengewinde 15 versehen, so daß eine Hutmutter 16, die ein entsprechendes Innengewinde 17 aufweist, aufschraubbar ist. Die Hutmutter 16 weist an ihrem unteren Ende einen Zentrierbund 18 auf, der das Aufschrauben der Hutmutter 16 auf das Gewinde 15 erleichtert. Koaxial zum Innengewinde 17 ist die Hutmutter 16 mit einem weiteren Innengewinde 19 versehen, welches einen kleineren Durchmesser aufweist. In dieses Innengewinde 19 ist eine Fixierschraube 20, die als Madenschraube ausgebildet ist, einschraubbar. Diese Fixierschraube 20 weist an ihrer Oberseite einen Innensechskant 21 auf und ist an ihrer Unterseite mit einer Ringschneide 22 versehen. Diese Ringschneide 22 gräbt sich beim Anziehen der Fixierschraube 20 in den Stab 10 ein. Die Oberseite der Hutmutter 16 sowie die Oberseite der Fixierschraube 20 ist ballig ausgeführt und die Hutmutter 16 weist einen Außensechskant 23 als Werkzeugsangriffsfläche auf, über die die Hutmutter 16 auf die Knochenschraube 5 aufgedreht und festgezogen werden kann.

Die in der Figur 1 rechts dargestellte Knochenschraube 6 weist ebenfalls einen gabelförmigen Schraubenkopf 8 auf, in den ein als Gewindestab 24 ausgebildeter Stab 9 eingelegt werden kann. Der Schraubenkopf 8 weist hierfür ebenfalls zwei Schenkel 25 und 26 auf, die zwischen sich eine Aufnahmeöffnung 27 bilden, in die der Gewindestab 24 eingelegt werden kann. Um die Aufnahmeöffnung 27 erstreckt sich eine kegelstumpfförmige Ausnehmung 28. In diese Ausnehmungen 28 des Schraubenkopfes 8 greifen Vorsprünge 29 ein, die an den Stirnseiten von Muttern 30, die auf den Gewindestab 24 aufgeschraubt sind, vorgesehen sind. Über diese Muttern 30 wird sowohl die axiale Lage des Gewindestabes im Schraubenkopf 8 der Knochenschraube 6 bestimmt als auch der Gewindestab 24 gegen ein axiales Verschieben, Verdrehen um dessen Achse und Herausrutschen aus dem Schraubenkopf 8 gesichert.

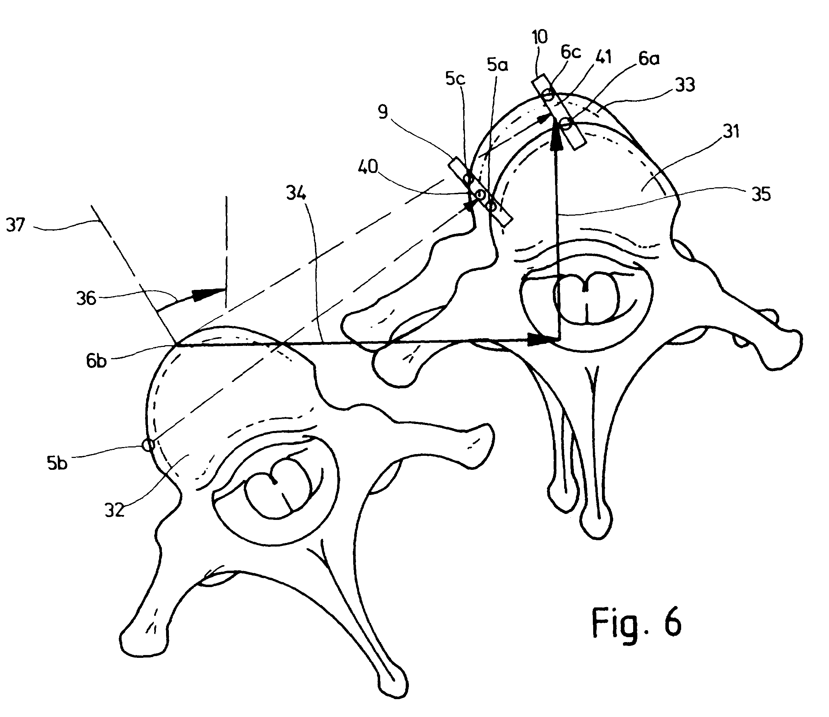

In der Figur 6 sind drei Wirbelkörper 31 bis 33 dargestellt, wobei der Wirbelkörper 32 eine Fehlstellung aufweist. Dieser Wirbelkörper 32 wird vom aktiven Stab 9 frontal in Richtung des Pfeiles 34 und sagital in Richtung des Pfeiles 35 verschoben und in Richtung des Pfeiles 36 gedreht (dreidimensionale Richtungsänderung). Durch die Translation wird der Wirbelkörper 32 zwischen die beiden Wirbelkörper 31 und 33 eingereiht und durch die Rotation ausgerichtet, so daß seine Hauptachse 37 parallel oder wenigstens annähernd parallel zu denen der Wirbelkörper 31 und 33 ausgerichtet ist. Mit den Bezugszeichen 5a-5c und 6a-6c sind die Knochenschrauben und mit dem Bezugszeichen 9 und 10 die Stäbe schematisch dargestellt. Nach der Korrektur befindet sich die Knochenschraube 5b und 6b bei 40 und 41, d.h. zwischen den Knochenschrauben 5a und 5c bzw. 6a und 6c. In dieser Lage werden die Wirbelkörper 31 bis 33 mit dem Stab 10 gegeneinander fixiert.

## Patentansprüche

1. Osteosynthesevorrichtung mit wenigstens zwei an einem Wirbel einer Wirbelsäule befestigbaren Knochenschrauben (5 und 6), wobei jede Knochenschraube (5 und 6) einen Schraubenkopf (7 und 8) aufweist, an dem bzw. mit dem ein Stab (9 bzw. 10) befestigt ist und die beiden Stäbe (9 und 10) über eine Querverbindungseinrichtung miteinander verbunden sind, wobei einer der Stäbe (10) als längsgenuteter, gerändelter oder glatter Stab ausgebildet ist, **dadurch gekennzeichnet**, daß der andere Stab (9) als aktiver, variabel verstellbarer Gewindestab (24) ausgebildet ist.

2. Osteosynthesevorrichtung nach Anspruch 1, dadurch gekennzeichnet, daß die Querverbindungseinrichtung als Knochenplatte (1) mit wenigstens zwei Befestigungsbohrungen (3 und 4) zur Aufnahme der beiden Knochenschrauben (5 und 6) ausgebildet ist.

3. Osteosynthesvorrichtung nach einem der vorhergehenden Ansprüche, dadurch gekennzeichnet, daß die Querverbindungseinrichtung als Querverbindungsstab ausgebildet ist.

4. Osteosynthesevorrichtung nach einem der vorhergehenden Ansprüche, dadurch gekennzeichnet, daß der Gewindestab (24) über zwei Muttern (30) am Schraubenkopf (8) der Knochenschraube (6) befestigbar ist.

5. Osteosynthesevorrichtung nach Anspruch 4, dadurch gekennzeichnet, daß der Schraubenkopf (8) in Achsrichtung des Gewindestabs (24) um die Aufnahmeöffnung (27) für den Gewindestab (24) sich erstreckende Vorsprünge und die beiden Muttern (30) die Vorsprünge aufnehmende Ausnehmungen aufweisen.

6. Osteosynthesevorrichtung nach Anspruch 4, dadurch gekennzeichnet, daß der Schraubenkopf (8) in Achsrichtung des Gewindestabs (24) um die Aufnahmeöffnung (27) für den Gewindestab (24) sich erstreckende Ausnehmungen (28) und die Muttern (30) in die Ausnehmungen (28) eingreifende Vorsprünge (29) aufweisen.

7. Osteosynthesevorrichtung nach Anspruch 5 oder 6, dadurch gekennzeichnet, daß die Ausnehmungen (28) und Vorsprünge (29) im wesentlichen kegelstumpfförmig, zylinderförmig oder kugelförmig ausgebildet sind.

8. Osteosynthesevorrichtung nach einem der vorhergehenden Ansprüche, dadurch gekennzeichnet, daß der Schraubenkopf (7 oder 8) gabelförmig ausgebildet ist.

9. Osteosynthesevorrichtung nach einem der vorhergehenden Ansprüche, dadurch gekennzeichnet, daß der Schraubenkopf (7) ein Außengewinde (15) für eine aufschraubbare Fixiereinrichtung aufweist.

10. Osteosynthesevorrichtung nach Anspruch 9, dadurch gekennzeichnet, daß die Fixiereinrichtung als Überwurfmutter oder als Hutmutter (16) ausgebildet ist.

11. Osteosynthesevorrichtung nach Anspruch 10, dadurch gekennzeichnet, daß die Hutmutter (16) mit einer koaxial einschraubbaren Fixierschraube (20), insbesondere mit einem Innensechskant (21) und/oder einer Ringschneide (22), versehen ist.

12. Osteosynthesevorrichtung nach einem der vorhergehenden Ansprüche, dadurch gekennzeichnet, daß der aktive Stab (9) ein Korrekturstab ist und der andere Stab (10) zur Stabilisierung, insbesondere zur Primärstabilisierung dient.

13. Osteosynthesevorrichtung nach einem der vorhergehenden Ansprüche, dadurch gekennzeichnet, daß die Befestigungsbohrungen (3 und 4) orthogonal zur Knochenplatte (1) oder schräg zu dieser verlaufen.

14. Osteosynthesevorrichtung nach einem der vorhergehenden Ansprüche, dadurch gekennzeichnet, daß die Befestigungsbohrungen (3 und 4) jeweils ein kalottenförmiges Lager für den Schraubenkopf (7 und 8) der Knochenschrauben (5 und 6) aufweisen.

15. Osteosynthesevorrichtung nach einem der vorhergehenden Ansprüche, dadurch gekennzeichnet, daß die beiden Stäbe (9 und 10) im wesentlichen parallel zueinander angeordnet sind.

16. Osteosynthesevorrichtung nach einem der vorhergehenden Ansprüche, dadurch gekennzeichnet, daß die Durchmesser der Stäbe (9 und 10) unterschiedlich sind, insbesondere der Durchmesser des Gewindestabs (24) kleiner als der des anderen Stabs (10) ist.

17. Osteosynthesevorrichtung nach einem der vorhergehenden Ansprüche, dadurch gekennzeichnet, daß der aktive Stab (9) eine größere Flexibilität als der andere Stab (10) aufweist.

18. Osteosynthesevorrichtung nach einem der vorhergehenden Ansprüche, dadurch gekennzeichnet, daß beide Stäbe (9 und 10) aus unterschiedlichen Materialien bestehen.

19. Osteosynthesevorrichtung nach einem der vorhergehenden Ansprüche, dadurch gekennzeichnet, daß sie zur ventralen Applikation geeignet ist.

20. Osteosynthesevorrichtung nach einem der vorhergehenden Ansprüche, dadurch gekennzeichnet, daß ein Stab (9) zur variablen Übertragung von im wesentlichen in Achsrichtung des Stabes wirkenden Zug- und Druckkräften ausgebildet ist.

21. Osteosynthesevorrichtung nach einem der vorhergehenden Ansprüche, dadurch gekennzeichnet, daß ein Stab (10) zur Übertragung von in der Orthogonalebene eines Stabs wirkenden Momenten ausgebildet ist und keine dynamischen Eigenschaften bezüglich der Wirbel besitzt.

22. Osteosynthesevorrichtung nach einem der vorhergehenden Ansprüche, dadurch gekennzeichnet, daß ein Stab (9) als dynamischer Teil und ein Stab (10) als statischer Teil der Osteosynthesevorrichtung ausgebildet ist.

23. Osteosynthesevorrichtung nach einem der vorhergehenden Ansprüche, dadurch gekennzeichnet, daß die Stäbe (9 bzw. 10) einen kreisrunden, ovalen , rechteckförmigen, dreieckförmigen oder einen anderen polygonförmigen Querschnitt oder eine Kombination hiervon aufweisen.

## Claims

1. An osteosynthesis device with at least two bone screws (5 and 6), which can be secured to a vertebra of a spinal column, wherein each bone screw (5 and 6) has a screw head (7 and 8) on which or with which a rod (9 or 10) is secured, and the two rods (9 and 10) are connected to each other via a lateral connecting device, wherein one of the rods (10) is embodies as as a rod which has longitudinal grooves, is milled, or is smooth, characterized in that the other rod (9) is embodied as an active, variably adjustable threaded rod (24).

2. The osteosynthesis device of claim 1, characterized in that the lateral connecting device is embodied as a bone plate (1) with at least two securing bores (3 and 4) for receiving the two bone screws (5 and 6).

3. The osteosynthesis device of one of the foregoing claims, characterized in that the lateral connecting device is embodied as a lateral connecting rod.

4. The osteosynthesis device of one of the foregoing claims, characterized in that the threaded rod (24) can be secured on the screw head (8) of the bone screw (6) via two nuts (30).

5. The osteosynthesis device of claim 4, characterized in that the screw head (8) has protrusions extending in the axial direction of the threaded rod (24) around the receiving opening (27) for the threaded rod (24) and the two nuts (30) have recesses which receive the protrusions.

6. The osteosynthesis device of claim 4, characterized in that the screw head (8) has recesses (28) extending in the axial direction of the threaded rod (24) around the receiving opening (27) for the threaded rod (24) and the nuts (30) have protrusions (29) which engage in the recesses (28).

7. The osteosynthesis device of claim 5 or 6, characterized in that the recesses (28) and protrusions (29) are embodied as essentially in the shape of truncated cones, cylinders, or balls.

8. The osteosynthesis device of one of the foregoing claims, characterized in that the screw head (7 or 8) is embodied as fork-shaped.

9. The osteosynthesis device of one of the foregoing claims, characterized in that the screw head (7) has an external thread (15) for a fixing device which can be screwed on.

10. The osteosynthesis device of claim 9, characterized in that the fixing device is embodied as a union nut or acorn nut (16).

11. The osteosynthesis device of claim 10, characterized in that the acorn nut (16) is provided with a fixing screw (20), which can be screwed in coaxially, in particular having an internal hex (21) and/or an annular cutting edge (22).

12. The osteosynthesis device of one of the foregoing claims, characterized in that the active rod (9) is a correction rod and the other rod (10) serves for stabilization, in particular primary stabilization.

13. The osteosynthesis device of one of the foregoing claims, characterized in that the securing bores (3 and 4) run orthogonal to the bone plate (1) or oblique to it.

14. The osteosynthesis device of one of the foregoing claims, characterized in that the securing bores (3 and 4) each have a ball-shaped bearing for the screw head (7 and 8) of the bone screws (5 and 6).

15. The osteosynthesis device of one of the foregoing claims, characterized in that the two rods (9 and 10) are disposed essentially parallel to each other.

16. The osteosynthesis device of one of the foregoing claims, characterized in that the diameters of the rods (9 and 10) are different: in particular the diameter of the threaded rod (24) is smaller than that of the other rod (10).

17. The osteosynthesis device of one of the foregoing claims, characterized in that the active rod (9) has a greater flexibility than the other rod (10).

18. The osteosynthesis device of one of the foregoing claims, characterized in that the two rods (9 and 10) are comprised of different materials.

19. The osteosynthesis device of one of the foregoing claims, characterized in that it is suited for ventral application.

20. The osteosynthesis device of one of the foregoing claims, characterized in that one rod (9) is embodied for variable transfer of tensile and compressive forces acting essentially in the axial direction of the rod.

21. The osteosynthesis device of one of the foregoing claims, characterized in that one rod (10) is embodied for transferring moments acting in the orthogonal plane of a rod and which has no dynamic properties with regard to the vertebrae.

22. The osteosynthesis device of one of the foregoing claims, characterized in that one rod (10) is embodied as the dynamic part and one rod (10) is embodied as the static part of the osteosynthesis device.

23. The osteosynthesis device of one of the foregoing claims, characterized in that the rods (9 or 10) have a circular, oval, rectangular, triangular, or other polygonal cross section or a combination thereof.

## Revendications

1. Dispositif d'ostéosynthèse comportant au moins deux vis pour os (5 et 6) susceptibles d'être fixées sur une vertèbre de colonne vertébrale, chaque vis pour os (5 et 6) présentant une tête de vis (7 et 8) sur ou avec laquelle est fixée une tige (9 ou 10), les deux tiges (9 et 10) étant associées par un dispositif de liaison transversal, l'une des deux (10) étant agencée sous forme d'une tige à rainure longitudinale, moletée ou lisse, caractérisé en ce que l'autre tige (9) est agencée sous forme d'une tige filetée (24) active, réglable de façon variable.

2. Dispositif d'ostéosynthèse selon la revendication 1, caractérisé en ce que le dispositif de liaison transversal est agencé sous forme d'une plaque d'ostéosynthèse (1) comportant au moins deux trous de fixation (3 et 4) pour la réception des deux vis pour os (5 et 6).

3. Dispositif d'ostéosynthèse selon l'une quelconque des revendications 1 et 2, caractérisé en ce que le dispositif de liaison transversal est agencé sous forme d'une tige de liaison transversale.

4. Dispositif d'ostéosynthèse selon l'une quelconque des revendications 1 à 3, caractérisé en ce que la tige filetée (24) peut être fixée au moyen de deux écrous (30) sur la tête de vis (8) de la vis pour os (6).

5. Dispositif d'ostéosynthèse selon la revendication 4, caractérisé en ce que la tête de vis (8) présente, dans l'axe de la tige filetée (24), des saillies entourant l'ouverture (27) recevant la tige filetée (24) et que les deux écrous (30) présentent des évidements recevant ces saillies.

6. Dispositif d'ostéosynthèse selon la revendication 4, caractérisé en ce que la tête de vis (8) présente, dans l'axe de la tige filetée (24), des évidements (28) entourant l'ouverture (27) recevant la tige filetée (24) et que les deux écrous (30) présentent des parties en saillie (29) pénétrant dans les évidements (28).

7. Dispositif d'ostéosynthèse selon l'une quelconque des revendications 5 ou 6, caractérisé en ce que les évidements (28) et saillies (29) présentent essentiellement une forme tronconique, cylindrique ou sphérique.

8. Dispositif d'ostéosynthèse selon l'une quelconque des revendications 1 à 7, caractérisé en ce que la tête de vis (7 ou 8) présente une forme de fourche.

9. Dispositif d'ostéosynthèse selon l'une quelconque des revendications 1 à 8, caractérisé en ce que la tête de vis (7) présente un filetage (15) pour un moyen de fixation vissable.

10. Dispositif d'ostéosynthèse selon la revendication 9, caractérisé en ce que le moyen de fixation est agencé sous forme d'écrou à raccord ou d'écrou borgne (16).

11. Dispositif d'ostéosynthèse selon la revendication 10, caractérisé en ce que l'écrou borgne (16 ) est muni d'une vis de fixation (20) à vissage coaxial, notamment d'une vis à six pans creux (21) et/ou une cuvette (22).

12. Dispositif d'ostéosynthèse selon l'une quelconque des revendications 1 à 11, caractérisé en ce que la tige active (9) est une tige de correction et que l'autre tige (10) sert à la stabilisation, notamment la stabilisation primaire.

13. Dispositif d'ostéosynthèse selon l'une quelconque des revendications 1 à 12, caractérisé en ce que les trous de fixation (3 et 4) se trouvent dans un plan orthogonal ou oblique par rapport à la plaque d'ostéosynthèse (1).

14. Dispositif d'ostéosynthèse selon l'une quelconque des revendications 1 à 13, caractérisé en ce que les trous de fixation (3 et 4) présentent chacun un logement sous forme de calotte pour la tête de vis (7 et 8) des vis pour os (5 et 6).

15. Dispositif d'ostéosynthèse selon l'une quelconque des revendications 1 à 13, caractérisé en ce que les deux tiges (9 et 10) sont disposées de manière essentiellement parallèle.

16. Dispositif d'ostéosynthèse selon l'une quelconque des revendications 1 à 15, caractérisé en ce que le diamètre des tiges (9 et 10) est différent, et que notamment le diamètre de la tige filetée (24) est inférieur à celui de l'autre tige (10).

17. Dispositif d'ostéosynthèse selon l'une quelconque des revendications 1 à 16, caractérisé en ce que la tige active (9) présente une plus grande flexibilité que l'autre tige (10).

18. Dispositif d'ostéosynthèse selon l'une quelconque des revendications 1 à 17, caractérisé en ce que les deux tiges (9 et 10) sont faites dans des matériaux différents.

19. Dispositif d'ostéosynthèse selon l'une quelconque des revendications 1 à 18, caractérisé en ce qu'il est adapté pour une application ventrale.

20. Dispositif d'ostéosynthèse selon l'une quelconque des revendications 1 à 19, caractérisé en ce qu'une tige (9) est aménagée pour permettre la transmission variable d'efforts de traction et de pression s'exerçant essentiellement en direction axiale de la tige.

21. Dispositif d'ostéosynthèse selon l'une quelconque des revendications 1 à 20, caractérisé en ce qu'une tige (9) est aménagée pour permettre la transmission de couples s'exerçant dans le plan orthogonal d'une tige et qu'elle ne possède pas de caractéristiques dynamiques par rapport aux vertèbres.

22. Dispositif d'ostéosynthèse selon l'une quelconque des revendications 1 à 21, caractérisé en ce qu'une tige (9) est aménagée comme partie dynamique et une tige (10) comme partie statique du dispositif d'ostéosynthèse.

23. Dispositif d'ostéosynthèse selon l'une quelconque des revendications 1 à 22, caractérisé en ce que les tiges (9 et 10) présentent une section circulaire, ovale, rectangulaire, triangulaire ou une autre section polygone ou une combinaison de celles-ci.
